(19) Europäisches Patentamt
European Patent Office
Office européen des brevets

(11) Veröffentlichungsnummer: **0 502 082 B1**

(12) **EUROPÄISCHE PATENTSCHRIFT**

(45) Veröffentlichungstag der Patentschrift: **08.11.95**

(51) Int. Cl.⁶: **A61L  27/00**

(21) Anmeldenummer: **91900182.6**

(22) Anmeldetag: **28.11.90**

(86) Internationale Anmeldenummer:
**PCT/DE90/00913**

(87) Internationale Veröffentlichungsnummer:
**WO 91/08000 (13.06.91 91/13)**

(54) **Gelenkendoprothese mit Al2O3-Keramikkopf und einer Kompositpfanne sowie Verfahre n zur Herstellung.**

(30) Priorität: **28.11.89 DD 334926**

(43) Veröffentlichungstag der Anmeldung:
**09.09.92 Patentblatt  92/37**

(45) Bekanntmachung des Hinweises auf die
Patenterteilung:
**08.11.95 Patentblatt  95/45**

(84) Benannte Vertragsstaaten:
**CH DE ES FR GB IT LI**

(56) Entgegenhaltungen:
**EP-A- 0 216 016**
**EP-A- 0 315 795**
**DE-B- 2 365 022**

**Chemical Abstracts, Band 98, no. 10, 29 April 1983, (Columbus, Ohio, US), Yuan T et al.:"Femoral Components (cup) made of glass-ceramics", siehe Seite 344**

(73) Patentinhaber: **ESKA MEDICAL LÜBECK ME-DIZINTECHNIK GmbH & Co.**
**Grapengiesserstrasse 21**
**D-23556 Lübeck (DE)**

(72) Erfinder: **NEUMANN, Gert**
**Galenusstrasse 23**
**D-1100 Berlin (DE)**
Erfinder: **BERGER, Georg**
**Rosenthaler Strasse 5**
**D-1113 Berlin (DE)**
Erfinder: **STEINBORN, Gabriele**
**Pichelsberger Strasse 3**
**D-1100 Berlin (DE)**
Erfinder: **LEUNER, Barbara**
**Bertolt-Brecht-Strasse 8**
**D-6325 Ilmenau (DE)**
Erfinder: **RAAB, Dagmar**
**Maumannstrasse 9**
**D-6300 Ilmenau (DE)**
Erfinder: **GLIEN, Willfried**
**Platz des 7. Oktober 3**
**D-6530 Hermsdorf (DE)**

Erfinder: **HUTSCHENREUTHER, Peter**
**Tachower Ring 1**
**D-6540 Stadtroda (DE)**


(74) Vertreter: **Dr. Fuchs, Dr. Luderschmidt Dr.**
**Mehler, Dipl.-Ing. Weiss Patentanwälte**
**Postfach 46 60**
**D-65036 Wiesbaden (DE)**

**Beschreibung**

Die Erfindung betrifft die Anwendung von glasig-kristallinen Materialien in der Gelenkendoprothetik durch eine elastische Einlagerung geeigneter Keramikkörper in physiologisch unbedenkliche Polyadditionskunststoffe pfannenseitig und die Minimierung der Reibungs- und Verschleißkräfte durch Kombination mit hochglanzpolierten keramischen Körpern kugelseitig bei optimaler Gestaltung der Gleitflächen zueinander. Der Kompositwerkstoff dient somit als Gleitpartner in künstlichen Gelenken, wie Hüft-, Knie-, Ellenbogen-, Schulter-, Hand-, Fußgelenkprothesen etc., bei denen der andere Gleitpartner aus einem Formkörper aus $Al_2O_3$-Keramik besteht. Derartige Prothesen finden in verschiedenen Disziplinen der Knochenersatzchirurgie, wie der Orthopädie und Traumatologie, ihren Einsatz.

Obwohl auf dem Gebiet des künstlichen Gelenkersatzes mit einem Gleitpartner, bestehend aus einer $Al_2O_3$-Keramik und dem anderen bestehend aus einem Kunststoff, Fortschritte erzielt wurden, sind hinreichende Beispiele von Prothesenversagen bekannt geworden.

Der dabei erzielte Stand bis etwa zum Jahr 1987 wurde von P. Eyerer dargestellt (in: Z. f. Werkstofftechnik 17 (1988) 384-91, 422-28, 444-48).

In der DD-PS 272603 wurden ferner die Nachteile von dem bislang umfangreich genutzten Polyethylen in seinen verschiedenen Varianten dargelegt (z.B. PS-D 2129832). Es wurde darin aber auch erläutert, warum man nicht auf die dämpfende Wirkung eines organischen Polymers bei Stoßbelastung z.B. in einer Hüftgelenkendoprothese verzichten sollte, wodurch der Einsatz einer Pfanne und eines Gelenkkopfes aus $Al_2O_3$-Keramik a priori in Frage zu stellen ist (DE-PS 2305333).

Bei Implantaten aus bioinerten Materialien auf Basis keramischer Werkstoffe besteht nämlich ein großer Modulunterschied zwischen Implantat und Knochen, der oftmals die Ursache für ein Implantatversagen darstellt.

Die damit verbundenen Versagensmechanismen beruhen einerseits auf ungedämpften Spannungsspitzen in der Gleitpaarung und folgendem Druck eines Gleitpartners und andererseits auf den ständigen lokalen Reizen im knöchernen Implantatlager und folgendem Knochenabbau.

Als Lösung dieser Probleme wurde deshalb ein Kompositwerkstoff vorgestellt, der sich auf ein vernetztes Polymer aufweisend insbesondere eine Polyurethan- oder Epoxidharzkomponente und auf einen Füllstoff gründet.

In zu diesem Komplex zählenden Erfindungsbeschreibungen wird ausgeführt, daß sich Werkstoffe von $CaO-P_2O_5-SiO_2$-Typ mit Apatit- und Wollastonitkristallphasen als vorteilhaft erweisen.

Andererseits ist gerade von diesen Stoffen bisher bekannt, daß sie hervorragende bioaktive Eigenschaften besitzen, d.h. infolge ihrer vorzüglichen Oberflächenreaktivität einen direkten (bindegewebsfreien) Knochenverbund eingehen.

Aus der EP-0216016 ist beispielsweise ein anorganisch organischer Verbundwerkstoff für biomedizinische Zwecke bekannt. Bei diesem Verbundwerkstoff gehen anorganische Ausgangsstoffe mit organischen Ausgangsstoffen eine neue feste chemische Verbindung ein, wobei die anorganische Ausgangskomponente ein biokompatibles Silikatglas und/oder eine Silikatglaskeramik des Systems $SiO_2-Al_2O_3-MgO-Na_2O/K_2O-F$ und/oder bioaktives Phosphorsilikatglas und/oder eine Phosphorsilikatglaskeramik des Systems $SiO_2-Al_2O_3-MgO-Na_2O/K_2O-CaO-P_2O_5-F$ und/oder des Systems $SiO_2-MgO-K_2O-F-CaO-P_2O_5$ darstellt und/oder die anorganische Ausgangskomponente ein bioaktives Phosphatglas und/oder eine Phosphatglaskeramik des Systems $P_2O_5-Al_2O_3-CaO-Na_2O/K_2O$ und/oder eine auf der Basis von Tricalziumphosphat und/oder Apatit beruhende Sinterkeramik darstellt und die organische Ausgangskomponente ein biokompatibler, monomerfreier, nur aus den Elementen C, H, O zusammengesetzter epoxydierter polymerer Kohlenwasserstoff mit einer mittleren Molmasse M von 2000 bis 6000 und Epoxidäquivalenten EÄG von 50 bis 500 g darstellt und die anorganische Ausgangskomponente ein phosphorsäuremodifizierter Stoff darstellt.

Obwohl diese Stoffe sich offenbar auch zur Herstellung des Kompositwerkstoffes als Gleitpartner für eine Komposit/$Al_2O_3$-Keramik-Paarung eignen, ist gerade durch die Oberflächenreaktivität der begründete Verdacht nicht auszuräumen, daß in Bezug auf diese Anwendung (ohne direkten Knochenkontakt) die Hydrolysestabilität zu gering ist. Schon allein aus Gründen der Prävention eines dadurch bedingten Implantatausfalls, sollte man nach einem Werkstoff suchen, der einerseits die hervorragenden Gleiteigenschaften mindestens beibehält oder gar verbessert und andererseits eine deutliche Hydrolysestabilität im Vergleich zu den bekannten, bioaktiven Werkstoffen (z.B. DD 248351 A1, JP-P 57/191252) aufweist.

Es ist Ziel der Erfindung, langzeitstabile und verschleißfeste Gelenkendoprothesen zu entwickeln.

Der Erfindung liegt die Aufgabe zugrunde, die Hydrolysestabilität bekannter Gelenkendoprothesen mit einer Pfanne aus einer polymeren Matrix und einer darin enthaltenen anorganischen Komponente mit Apatit- und/oder Wollastonit-Kristallphasen zu verbessern.

Die Aufgabe der Erfindung wird dadurch erreicht, daß bei Einsatz als konvexen Teil des Kunstgelenks einer bekannten $Al_2O_3$-Keramik mit hochglanzpolierter Oberfläche ein verschleißfreier Lauf dieses konvexen Teils durch eine Einbettung von hochfesten mineralischen Abrasionspräventoren in der Lauffläche der Gelenkpfanne erreicht wird. Diese Einbettung erfolgt so, daß von vornherein eine große Zahl von Berührungspunkten zwischen den Reibpartnern durch die mineralischen Komponenten gebildet wird und daß die Zahl der Berührungspunkte auch bei deformierender Belastung des konkaven Teils des Kunstgelenks möglichst groß bleibt. Die eingebetteten Abrasionspräventoren besitzen darüber hinaus eine hohe Affinität zum Einbettmaterial, so daß die auftretenden Reibungskräfte keine Teilchen aus der Lauffläche herauszulösen vermögen. Dies wird erfindungsgemäß durch einen optimalen Öffnungswinkel beziehungsweise durch eine optimale Differenz zwischen Innen- und Außenkugel einerseits und andererseits durch die optimale Korngrößenverteilung der Abrasionspräventoren erreicht.

Erfindungsgemäß variiert die Zusammensetzung des die Lauffläche der Pfanne bildenden glaskeramischen Materials für folgende Bestandteile jeweils (Angaben in Masse-%)

| | |
|---|---|
| CaO | 31 - 34 |
| $P_2O_5$ | 10 - 12 |
| $SiO_2$ | 43 - 46 |
| $Na_2O$ | 3 - 5 |
| MgO | 2 - 3,5 |
| $CaF_2$ | 3 - 5 |

und stets zusätzlich, über die 100% der vorgenannten Gemengebestandteile hinausgehend, weitere 0,5 bis 11% an $Al_2O_3$ und/oder $ZrO_2$ oder $TiO_2$ und $Al_2O_3$ oder $TiO_2$ und $ZrO_2$ oder $TiO_2$ und $ZrO_2$ und $Al_2O_3$ enthält, wobei $TiO_2$ zwischen 0,5 bis 5% liegt.

Die genannte Differenz zwischen Innenradius der Pfanne und Außenradius des Kopfes liegt erfindungsgemäß zwischen 25 und 350 Mikrometer. Diese Differenz ist deshalb erfindungswesentlich, weil eine sogenannte Einlaufphase wie bei Polyethylenpfannen bei der erfindungsgemäßen Endoprothese nicht auftritt und von vornherein dieser Öffnungswinkel bzw. diese Differenz festzulegen ist. Sie beträgt vorzugsweise 30 bis 300 Mikrometer. Pfanne und Kugel sollen möglichst eine ideale Kugelform aufweisen, d.h. Radiusabweichungen von mehr als 30 Mikrometer sind nicht zu tolerieren.

Weiterhin ist es vorteilhaft, daß die anorganische Kompositkomponente Nebenkristallphasen von 1 bis 20% enthält, ausgwählt aus der Gruppe Feldspate, alpha-Tricalciumphosphat, $CaTiSiO_5$ und Zirkonate. Die Menge der Nebenkristallphasen beträgt vorzugsweise 5 - 20%, insbesondere 8 - 15%.

Eine bevorzugte Zusammensetzung der organischen Komponente enthält zusätzlich zu 100% Gemenge 0,5 - 10% $ZrO_2$. Eine weitere bevorzugte Zusammensetzung enthält zusätzlich 0,5 - 5% $TiO_2$ und $Al_2O_3$ und/oder $ZrO_2$ bis insgesamt 10%. Eine weitere bevorzugte Zusammensetzung enthält zusätzlich 0,5 bis 10% $Al_2O_3$. Eine letzte bevorzugte Zusammensetzung enthält zusätzlich 0,5 - 10% $ZrO_2$ und $Al_2O_3$.

Gegenstand der Erfindung ist ferner ein Herstellungsverfahren einer Gelenkendoprothese mit $Al_2O_3$-Keramikkopf und einer Kompositpfanne durch Schmelzen eines anorganischen Gemenges, Abkühlen und Zerkleinern des kristallinen Materials mit Apatit- und /oder Wollastonitkristallphasen, Einbringen in eine polymere Matrix und Aushärten der Matrix in einer Pfannenform. Das Verfahren ist gekennzeichnet durch die obige Gemengezusammensetzung, das o.g. Korngrößenspektrum, das in der polymeren Matrix verteilt wird und die Aushärtung in einer Pfanne mit einem derart gehaltenen Innenradius, daß die spätere Kombination mit dem $Al_2O_3$-Keramikkopf nur eine Differenz zwischen Innenradius der Pfanne und Außenradius der Kugel im Bereich von 25 bis 350 Mikrometer zuläßt.

Als polymere Matrix kann ein Polyurethan oder ein Epoxidharz eingesetzt werden. Diese sind in einem Anteil von 20 bis 60 Masseteile in % vorhanden. Vorteilhaft bei Einsatz eines Polyurethanes ist eine Polyfunktionalität zwischen 2,1 und 4,0 insbesondere 3,3 - 3,6.

Insgesamt soll der Anteil der funktionellen Gruppen bei den Polymeren gleich oder größer 10% sein und die Glastemperatur oberhalb der Körpertemperatur liegen. Die Gelierzeit beträgt wenigstens 3 Minuten.

Im Sinne der Erfindung ist es gleichgültig nach welchem Verfahren glasig-kristallines Material hergestellt wurde, d.h., ob es sich um eine Glaskeramik, eine Keramik oder eine Sinterglaskeramik handelt. Die Herstellungsvariante nach der Glaskeramik-Technologie scheint infolge leicht verbesserter Biegebruchfestigkeitswerte des nicht gekörnten Materials bevorzugt das Erfindungsziel zu gewährleisten.

Die Anwesenheit von Apatit und/oder Wollastonit ist beim erfindungsgemäßen Material unerläßlich. Die Versuche haben aber auch zu dem Ergebnis geführt, daß Nebenkristallphasen, wie z.B. Feldspate, alpha-Tricalciumphosphat, $CaTiSiO_5$, verschiedene Zirkonate etc. nicht wie erwartet nur in einem eng begrenzten Maße toleriert werden, sondern überraschend konnte festgestellt werden, daß ihre Anwesenheit sich als günstig erweisen kann bis zu einem beachtlichen Mengenanteil.

Dennoch kann der Anteil der Nebenphasen einen bestimmten Anteil nicht überschreiten. Daher sollte der maximale Masse-%-Anteil kleiner gleich 15 betragen, wenn eine weitere Nebenkristallphase neben den Hauptkristallphasen vorliegt, und maximal sollte er 20 betragen, wenn zwei oder mehrere Nebenkristallphasen vorhanden sind. Es versteht sich von selbst, daß die Hauptkristallphase bzw. -phasen stets einen höheren Mengenanteil bilden als die Nebenkristallphasen.

Zur Charakterisierung eines weiteren Merkmales des glasig-kristallinen Materials wurde eine Schnellmethode zur Bestimmung der chemischen Stabilität herangezogen.

Das zu untersuchende Material wird zerkleinert und die für die Bestimmung gewählte Kornfraktion von 315 - 400 $\mu$m wird entnommen. Das Probematerial wird mit Ethanol gewaschen und anschließend bei 110° C getrocknet. Es werden 10 Proben von jeweils ca. 2 g der Untersuchungssubstanz auf der Analysenwaage bestimmt. Bidestilliertes Wasser wird auf 37° C erhitzt und jeweils 200 ml im Becherglas werden mit den eingewogenen ca. 2 g versetzt. Diese Probe bleibt 24 h im Brutschrank bei 37° C, abgedeckt mit einem Uhrglas, stehen. Nach dieser Zeit werden die Proben in vorher ausgewogene Filter überführt, die Bechergläser werden dabei mit destilliertem Wasser ausgespült bis nichts mehr zurückbleibt. Danach werden die Filter mit der Probensubstanz bei 110° C getrocknet. Nach dem Abkühlen im Exsikkator erfolgt die erneute Wägung zur Ermittlung des Gewichtsverlustes nach: (Einwaage in mg - Auswaage in mg) * 1000/Einwaage in mg = Ergebnis in mg Substanzverlust/g Einwaage).

Ein Teil der danach bestimmten Ergebnisse wurde zusammen mit Angaben zur Kristallphasenbestimmung und der chemischen Zusammensetzungen von relevanten Material und von Vergleichsbeispielen A und B in Tabelle 1 aufgeführt.

Es zeigt sich, daß im Vergleich zu einigen Materialien des Grundsystems $P_2O_5$-$CaO$-$SiO_2$ die hier beschriebenen Materialien C und D chemisch hydrolysestabiler sind.

Die Erfindung wird weiterhin dadurch gekennzeichnet, daß das Kornband des verwendeten keramischen Materials einen Anteil mit einer Korngröße kleiner 10 $\mu$m von weniger als 0,1% aufweist. Optimal erweist sich folgendes Vorgehen:

Ein glasig-kristallines Material wird bei 150° C in dünner Schicht vorgetrocknet, nachdem zovor ein Kornband von 50 - 500 $\mu$m, vorteilhafterweise jedoch nur 63 - 200 $\mu$m bzw. besonders bevorzugt nur 100 - 200 $\mu$m zusammengestellt wurde. Dieses Kornband sollte sich folgendermaßen zusammensetzen:

| | |
|---|---|
| 200 bis 500 $\mu$m | 10% |
| 150 bis 200 $\mu$m | 15 - 25% |
| 125 bis 160 $\mu$m | 40 - 50% |
| 100 bis 125 $\mu$m | 20 - 30% |
| 100 $\mu$m | 20% |

Wird das Kornband enger gewählt, erhöht sich folglich der Anteil der ausgewählten Fraktionen, was in obiger Zusammenstellung berücksichtigt wurde. Aus den Angaben ist zu entnehmen, daß es sich nicht um ein normalverteiltes Partikelhaufwerk handelt.

Für einen verschleißfreien Lauf des Kunstgelenks ist ein Anteil von 20 bis 60 Masseanteile in % (bezogen auf die Gesamtkompositmasse) erforderlich.

Überraschenderweise wurde gefunden, daß durch einfaches Einführen derartiger so charakterisierter grobkristalliner, harter Abrasionspräventoren in eine flüssige Harzmischung auf der Basis eines geeigneten Polyadditionskunstharzes, das der Forderung nach physiologischer Unbedenklichkeit gehorcht, und durch Vergießen dieses flüssigen Kristallbreis in eine entsprechende Form, eine Anordnung der Kristalloberflächen an der Oberfläche des resultierenden Formkörpers erreicht werden kann, die einen minimalen Verschleiß resultieren läßt.

Erfindungsgemäß sind Polyadditionskunststoffe, wie Epoxidharze und Polyurethane zur Herstellung von in der Medizintechnik einsetzbaren Formstoffen, die auch als Biomaterial bezeichnet werden, als besonders geeignet einzuschätzen, da zu deren Herstellung keinerlei Initiatoren, Beschleuniger, Weichmacher, Stabilisatoren und ähnliche später migrierende und teilweise toxische Zusätze notwendig sind. Darüberhinaus erfolgt die Verarbeitung beim erfindungsgemäßen Vorgehen lösungsmittelfrei. Zur Herstellung der Polyurethane werden einerseits niedermolekulare Hydroxylverbindungen wie Trimethylolpropan, Neopentylglykol, Hexandiol-1.6 und Butandiol-1.4 neben den höhermolekularen Polyolen wie Polytetrahydrofuran, oder Monorizinoleate der niedermolekularen Hydroxylverbindungen eingesetzt.

EP 0 502 082 B1

Als Additionskomponente werden 95 bis 105 Äquivalent% eines aromatischen, aliphatischen oder alicyclischen Diisocyanats eingesetzt. Als solche dienen beispielsweise die bekannten chemischen Verbindungen wie Hexamethylendiisocyanat, 4,4-Diphenylmethandiisocyanat oder das Gemisch aus 2,4 und 2,6-Toluylendiisocyanat.

Zur Gewährleistung der hydrolytischen Stabilität der Polymermatrix und zur festen Bindung der Abrasionspräventoren ist es wichtig, daß die Glastemperatur des zur Einbettung benutzten Gießharzes oberhalb der Körpertemperatur liegt und daß der Gehalt an funktionellen Gruppen, die eine feste Bindung zwischen Gießharz und Keramik bewirken mindestens 10% beträgt.

Als solche Gruppen fungieren in Polyurethanen die zur Wasserstoffbrückenbindung befähigten Urethangruppen, während in Epoxidharzen die bei Verwendung von Polyaminen als Härter gebildeten Hydroxylgruppen, bzw. bei Verwendung von Anhydriden gebildeten Estergruppen derartige Wechselwirkungen ausüben.

Zur Erreichung der erfindungsgemäß notwendigen Glastemperatur bei Polyurethan-Gießharzen ist es notwendig, die Polyolfunktionalität zwischen 2,1 und 4,0, vorzugsweise zwischen 3,3 und 3,6 einzustellen. Darüber hinaus ist es aus gleichem Grunde notwendig, daß das Hydroxyläquivalentgewicht des Polyols zwischen 100 g und 300 g Komponente/Mol OH vorzugsweise auf 150 g/Mol bis 220 g/Mol eingestellt wird.

Bei Epoxidharzen wird die Glastemperatur durch Verwendung von bekannten Diglycidethern der aromatischen Diphenole oder auch durch Verwendung von Isocyanursäuretriglycideester sowie durch Einsatz von Phthalsäureanhydrid, Hexahydrophthalsäureanhydrid und ähnlichen üblichen Epoxidharzhärter erreicht. Durch dieses Herstellungsverfahren, der Gießtechnik von beispielsweise Pfannen, werden wesentlich geringere arithmetrische Mittenrauhwerte und verbesserte Rundheiten erhalten, als es bei den traditionellen Herstellungsverfahren, der mechanischen (Nach-) Bearbeitung, überhaupt möglich ist.

Man erhält somit Gelenkendoprothesen, bestehend aus einem Gleitpartner aus $Al_2O_3$-Keramik, einen biokompatiblen, hydrolysestabilen Kompositwerkstoff als zweiten Gleitpartner der hochfest und insbesondere verschleißarme Gelenke liefert. Die erzielten Vorteile der verbesserten Hydrolysestabilität minimieren weiterhin das Restrisiko des Einsatzes derartiger Gelenkprothesen erheblich, besonders unter dem Aspekt des Langzeiteinsatzes im Fachbereich der Humanmedizin. Es wurde überraschenderweise festgestellt, daß ein ganz bestimmtes Kornspektrum für den "Füllstoff" existiert, das die optimalen Gleiteigenschaften garantiert. Dies ist natürlich wiederum eine Funktion des eingesetzten Materials. Der Begriff "Füllstoff" ist in dem Sinne irreführend, sofern davon abgeleitet werden sollte, daß zwischen dem organischen Polymeren und dem eingelagerten Material keine Wechselwirkungen eintreten würden.

Mit der Erfindung wurden ferner Materialien bereitgestellt, die zwar chemisch gleich, jedoch nach verschiedenen Verfahren hergestellt werden, um einerseits jeweils eine kostengünstige Herstellungsmethode auswählen zu können, um andererseits jedoch auch die verfahrenstechnisch bedingten Eigenschaftsmodifizierungen entsprechend den Gegebenheiten selektieren zu können. Damit wird es möglich, den einzelnen Einsatzgebieten, wie Finger- oder Hüftgelenkprothesen, entsprechend angepaßte Komposite zur Anwendung zu bringen Diese Eigenschaftsunterschiede wirken sich auch auf die Gesamteigenschaften aus, da Wechselwirkungen mit dem organischen Polymeren bestehen.

Bezogen auf die anorganische Komponente des Komposits wurde aus der Fülle der denkbaren Zusammensetzungen des Grundsystems $P_2O_5$-CaO-$SiO_2$ mit Zusätzen von $Al_2O_3$, $ZrO_2$ und $TiO_2$ diejenigen ausgewählt, die besonders hydrolysestabil sind und in denen der Komposit in der oben genannten Gleitpaarung zu keinem die Funktion beeinträchtigenden Verschleiß führt.

Die folgenden Beispiele erläutern die Erfindung.

Ausführungsbeispiel

Beispiel 1 bis 6 (A bis F)

Es wurden Gemenge gemäß Tabelle 1 geschmolzen und mittels üblicher Temperaturbedingungen in eine Keramik, Sinterglaskeramik oder Glaskeramik überführt.

6

Tabelle 1

| Beispiele für das glasig-kristalline Material | | | | | | |
|---|---|---|---|---|---|---|
| Komponente | Code der Zusammensetzung | | | | | |
| | A | B | C | D | E | F |
| | Vergleichsbeispiele | | | | | |
| CaO | 31,9 | 31,9 | 31,9 | 31,9 | 31,9 | 31,9 |
| $P_2O_5$ | 11,2 | 11,2 | 11,2 | 11,2 | 11,2 | 11,2 |
| $SiO_2$ | 44,5 | 44,5 | 44,5 | 44,5 | 44,5 | 44,5 |
| $Na_2O$ | 4,6 | 4,6 | 4,6 | 4,6 | 4,6 | 4,6 |
| MgO | 2,8 | 2,8 | 2,8 | 2,8 | 2,8 | 2,8 |
| $CaF_2$ | 4,8 | 5,0 | 5,0 | 5,0 | 5,0 | 5,0 |
| $K_2O$ | 0,2 | - | - | - | - | - |
| $TiO_2$ | - | 10,0 | - | - | - | - |
| $ZrO_2$ | - | - | 1,0 | 8,0 | 10,0 | - |
| $Al_2O_3$ | - | - | 1,0 | 1,0 | - | 10,0 |
| Haupt Kristallphasen | Ap  Wo | Ap  Wo | Ap  Wo | Ap  Wo | Ap  Wo | Ap  Wo |
| Nebenkristallphasen | - | $CaTiSiO_5$ Rutil | nicht identifizierbar | | $CaZr-Si_4-O_{12}$ | Feldspate, $\alpha$-TCP |
| Schnellmethode [mg/g] | 2,27 ± 0,8 | 2,26 ± 0,3 | 1,80 ± 0,2 | n.b. | 1,78 ± 0,1 | 1,76 ± 0,1 |
| Legende: Ap - Apatit; Wo - Wollastonit; TCP - Tricalciumphosphat; n. b. - nicht bestimmt | | | | | | |

Beispiel 7

20 g eines durch Vermischen eines Ricinolsäuretrimethylpropanesters mit weiterem Trimethylolpropan entstandenen Polyolgemischs mit dem Hydroxyläquivalentgewicht 190 g Ester/Mol OH und einer Funktionalität von 3,0 werden mit 20 g eines unten charakterisierten glaskeramischen Granulats innig gemischt und getrocknet. Bei 40° C besitzt die Polyolmischung eine Viskosität 1000 mPa.s. Zu dieser Suspension werden 10 g destilliertes Toluylendiisocyanat (Äquivalentgewicht 90 g/Mol NCO) gegeben und so eine bei 40° C gießfähige, härtbare Mischung hergestellt. Durch Eingießen in eine Form mit einer aufgerichteten Halbkugel und nach Aushärtung bei Temperaturen zwischen 40 und 80° C läßt sich aus der Form ein Pfannenrohling entformen, dessen Verschleißfestigkeit geprüft werden kann.

Als Bioglaskeramik wurde ein Material der chemischen Zusammensetzung entsprechend Beispiel C in folgender Kornfraktion für die Kompositbildung verwendet:

| | |
|---|---|
| bis 200 $\mu$m | 17,7% |
| bis 160 $\mu$m | 42,6% |
| bis 125 $\mu$m | 22,6% |
| bis 100 $\mu$m | 6,8% |
| bis 90 $\mu$m | 8,5% |
| bis 71 $\mu$m | 1,3% |
| <63 $\mu$m | <0,1% |

Die Verschleißuntersuchungen brachten folgende Ergebnisse: Ein gemäß oben zusammengesetztes Polyurethan kombiniert mit 40% Biokeramik zeigt in Versuchen gegen $Al_2O_3$-Keramik ein besseres Verschleißverhalten als PE, wie die Übersicht zeigt:

| Übersicht zu Verschleißverhalten | | | |
|---|---|---|---|
| | $Al_2O_3$/PE | $Al_2O_3$/Pfanne gem. Beispiel 7 | $Al_2O_3$/Pfanne gem. Beispiel 8 |
| 1. mg/Abrieb | 95 | nicht meßbar | nicht meßbar |
| 2. Arithmetische Rauigkeit, Durchschnitt in $\mu$m | 0,69 | 0,18 | 0,16 |
| 3. Max. Rauigkeit - Durchschnitt | 4,56 (8,82) x) | 2,16 (2,29) x) | 1,55 (2,00) x) |

x) Absolute maximale Rauigkeit Meßbedingungen: Reibversuche mit Wasser bei 37 ° C, 2000 N Last, 2 000 000 Lastwechsel, Prüffrequenz 1 Hertz, Schwenkwinkel 45 °

Beispiel 8

14 g eines kristallinen Epoxidharzes auf der Basis von Isocyanursäure werden mit 20 g frisch destilliertem Hexahydrophthalsäureanhydrid bei 110° C zur Polyaddition gebracht. Nach 20 minütiger Vorreaktion besitzt die Mischung eine Viskosität von etwa 500 mPa.s. Bei dieser Viskosität werden 20 g einer ausgewählten Bioglaskeramik eingerührt und die Suspension in die auf 100° C vorgeheizte Form gegossen. Nach dem Aushärten (24 h bei 110° C) kann ebenfalls ein Pfannenrohling entformt werden, der die in der Übersicht im Beispiel 7 gezeigten Verschleißeigenschaften hat.

Dabei wurde ein Material der chemischen Zusammensetzung entsprechend Beispiel C in folgender Kornfrakton für die Kompositbildung verwendet:

| bis 200 $\mu$m | 20,1% |
|---|---|
| bis 160 $\mu$m | 48,2% |
| bis 125 $\mu$m | 25,3% |
| bis 100 $\mu$m | 6,4% |

Die aufgeführten Eigenschaften zeigen die Eignung des beschriebenen erfindungsgemäßen Kompositmaterials als verschleißfestes Pfannenmaterial für Gelenkprothesen.

**Patentansprüche**

**1.** Gelenkendoprothese mit $Al_2O_3$-Keramikkopf und einer Kompositpfanne, deren anorganische Komponente Apatit- und/oder Wollastonit-Kristallphasen aufweist und deren organische Komponente ein vernetztes Polymeres auf Polyurethan- oder Epoxidbasis ist, wobei die anorganische Kompositkomponente eine Keramik, Glaskeramik oder Sinterglaskeramik ist, und das gebrannte Material als Partikel mit einer definierten Korngrößenverteilung in der Kompositpfanne verteilt ist, wobei die Differenz von Innenradius der Pfanne und Außenradius des Kopfes im Bereich von 25 bis 350 Mikrometer liegt und Pfanne und Kopf im wesentlichen ideale Kugelform aufweisen,
dadurch gekennzeichnet, daß
1.) die Keramik, Glaskeramik oder Sinterglaskeramik vor dem Brennen die Gemengezusammensetzung (Masseteile in %)

| CaO | 31 - 34 |
|---|---|
| $P_2o_5$ | 10 - 12 |
| $SiO_2$ | 43 - 46 |
| $Na_2O$ | 3 - 5 |
| MgO | 2 - 3,5 |
| $CaF_2$ | 3 - 5 |

aufweist und stets zusätzlich, über die 100% der vorgenannten Gemengebestandteile hinausgehend, weitere 0,5 bis 11% an $Al_2O_3$ und/oder $ZrO_2$ oder $TiO_2$ und $Al_2O_3$ oder $TiO_2$ und $ZrO_2$ oder $TiO_2$ und $ZrO_2$ und $Al_2O_3$ enthält, wobei $TiO_2$ zwischen 0,5 bis 5% betragen kann,
2.) die Korngrößenverteilung von der Gaußverteilung abweicht, wobei der Anteil der Partikel von

| 200 - 500 Mikrometer | kleiner 10% |
| 160 - 200 Mikrometer | 15 bis 25% |
| 125 - 160 Mikrometer | 40 bis 50% |
| 100 - 125 Mikrometer | 20 bis 30% |
| kleiner 100 Mikrometer | |
| kleiner 20% | |
| davon kleiner 10 Mikrometer | |
| kleiner 0,1% | |

beträgt, und

3.) die Radiusabweichungen von der Kugelform bei Pfanne und Kopf $\leq$ 30 $\mu$m sind.

2. Gelenkendoprothese nach Anspruch 1, dadurch gekennzeichnet, daß die anorganische Kompositkomponente Nebenkristallphasen in einer Menge von 1 bis 20 Masseteile in % enthält, wobei die Nebenkristallphasen aus der Gruppe ausgewählt sind, die aus Feldspaten, alpha-Tricalciumphosphat, $CaTiSiO_5$, $CaZrSi_4O_{12}$ und Rutil besteht.

3. Gelenkendoprothese nach Anspruch 2, dadurch gekennzeichnet, daß die anorganische Kompositkomponente Nebenkristallphasen in einer Menge von 5 - 20; vorzugsweise 8 bis 15 % enthält.

4. Gelenkendoprothese nach Anspruch 1, dadurch gekennzeichnet, daß das Gemenge zusätzlich $ZrO_2$ in einer Menge von 0,5 bis 10% enthält.

5. Gelenkendoprothese nach Anspruch 1, dadurch gekennzeichnet, daß das Gemenge zusätzlich 0,5 bis 5% $TiO_2$ und weiterhin $Al_2O_3$ und/oder $ZrO_2$ bis insgesamt 10% enthält.

6. Gelenkendoprothese nach Anspruch 1, dadurch gekennzeichnet, daß das Gemenge zusätzlich $Al_2O_3$ in einer Menge von 0,5 bis 10% enthält.

7. Gelenkendoprothese nach Anspruch 1, dadurch gekennzeichnet, daß das Gemenge zusätzlich $ZrO_2$ und $Al_2O_3$ in einer Menge von 0,5 bis 10% enthält.

8. Verfahren zur Herstellung einer Gelenkendoprothese mit $Al_2O_3$-Keramikkopf und einer Kompositpfanne durch
   a) Schmelzen eines anorganischen Gemenges zu einem keramischen, glaskeramischen oder sinterglaskeramischen Material,
   b) Abkühlen und Zerkleinern des kristallinen Materials mit Apatit- und/oder Wollastonit-Kristallphasen,
   c) Einbringen des kristallinen Materials aufweisend eine definierte Korngrößenverteilung in eine polymere Matrix auf Polyurethan- oder Epoxidbasis,
   d) Aushärten der Matrix in einer Pfannenform,
   e) und Kombination mit einem $Al_2O_3$-Keramikkopf, dessen Außenradius so gestaltet ist, daß die Differenz zwischen Innenradius der hochglanzpolierten Pfanne und Außenradius des Kopfes im Bereich von 25 bis 350 Mikrometer liegt,
   dadurch gekennzeichnet, daß
   A) das Gemenge folgende Zusammensetzung aufweist (Masseteile in %)

| CaO | 31 - 34 |
|---|---|
| $P_2O_5$ | 10 - 12 |
| $SiO_2$ | 43 - 46 |
| $Na_2O$ | 3 - 5 |
| MgO | 2 - 3,5 |
| $CaF_2$ | 3 - 5 |

und stets zusätzlich, über die 100% der vorgenannten Gemengebestandteile hinausgehend, weitere 0,5 bis 11% an $Al_2O_3$ und/oder $ZrO_2$ oder $TiO_2$ und $Al_2O_3$ oder $TiO_2$ und $ZrO_2$ oder $TiO_2$ und $ZrO_2$ und $Al_2O_3$ enthält, wobei $TiO_2$ zwischen 0,5 bis 5% betragen kann,

B) das damit hergestellte Material mit einer Korngrößenverteilung von

| 200 - 500 Mikrometer | kleiner 10% |
|---|---|
| 160 - 200 Mikrometer | 15 bis 25% |
| 125 - 160 Mikrometer | 40 bis 50% |
| 100 - 125 Mikrometer | 20 bis 30% |
| kleiner 100 Mikrometer | kleiner 20%, davon |
| kleiner 10 Mikrometer | kleiner 0,1% |

in der polymeren Matrix verteilt wird,

C) und die Radiusabweichung von der Kugelform sowohl bei der Pfanne als auch der Kugel $\leq 30$ $\mu$m sind.

9. Verfahren nach Anspruch 8, dadurch gekennzeichnet, daß das Gemenge folgende Zusammensetzung in Masse% aufweist

| CaO | 31 - 34 |
|---|---|
| $P_2O_5$ | 10 - 12 |
| $SiO_2$ | 43 - 46 |
| $Na_2O$ | 3 - 5 |
| MgO | 2 - 3,5 |
| $CaF_2$ | 3 - 5 |

und stets zusätzlich, über die 100 Masse-% der vorgenannten Gemengebestandteile hinausgehend, weitere 5 bis 10 Masse-% an $Al_2O_3$ und/oder $ZrO_2$ oder $TiO_2$ und $Al_2O_3$ oder $TiO_2$ und $ZrO_2$ oder $TiO_2$ und $ZrO_2$ und $Al_2O_3$ enthält, wobei $TiO_2$ zwischen 0,5 bis 5 Masse-% betragen kann.

10. Verfahren nach Anspruch 9, dadurch gekennzeichnet, daß das Gemenge zusätzlich 0,5 bis 5% $TiO_2$ und weiterhin $Al_2O_3$ und/oder $ZrO_2$ bis insgesamt 10% enthält.

11. Verfahren nach Anspruch 9, dadurch gekennzeichnet, daß dem Gemenge zusätzlich $Al_2O_3$ und/oder $ZrO_2$ zugesetzt wird in gleichen oder unterschiedlichen Mengenanteilen in den Grenzen von 0,5 bis 10 Masse-%.

12. Verfahren nach Anspruch 8, bei dem als polymere Matrix ein Polyurethan eingesetzt wird, hergestellt aus einem Polyol oder einem Gemisch aus verschiedenen Polyolen und einem aromatischen, aliphatischen oder alicyclischen Diisocyanat, oder bei dem die polymere Matrix aus einem Epoxidharz und einem Härtungsmittel gebildet wird, wobei die anorganische Komponente in die Harz-Härter-Mischung eingebracht wird, dadurch gekennzeichnet, daß der Anteil der funktionellen Gruppen gleich oder größer als 10% ist.

13. Verfahren nach Anspruch 12, dadurch gekennzeichnet, daß die Glastemperatur der polymeren Matrix oberhalb der Körpertemperatur liegt.

**14.** Verfahren nach Anspruch 12, dadurch gekennzeichnet, daß beim Vorliegen eines Polyurethans als polymerer Matrix die Polyfunktionalität zwischen 2,1 und 4,0, vorzugsweise 3,3 und 3,6 liegt.

**15.** Verfahren nach Anspruch 8 bis 14, dadurch gekennzeichnet, daß der Anteil des anorganischen Materials im Bereich von 20 bis 60 Masseteile in % liegt, bezogen auf die Gesamtkompositmasse.

**16.** Verfahren nach Anspruch 8, dadurch gekennzeichnet, daß die Gelierzeit der polymeren Komponente wenigstens 3 Minuten beträgt.

**Claims**

**1.** Joint endoprosthesis with $Al_2O_3$ ceramic head and a composite socket, the inorganic component of which has apatite and/or wollastonite crystal phases, and the organic component of which is a crosslinked polymer based on polyurethane or epoxide, wherein the inorganic composite component is a ceramic, glass ceramic or sintered glass ceramic, and the fired material is distributed as particles having a defined grain size distribution in the composite socket, wherein the difference between the internal radius of the socket and the external radius of the head is in the range from 25 to 350 micrometres and socket and head have substantially ideal ball shape, characterised in that
1.) the ceramic, glass ceramic or sintered glass ceramic before firing has the mixture composition (parts by mass in %)

| | |
|---|---|
| CaO | 31-34 |
| $P_2O_5$ | 10 - 12 |
| $SiO_2$ | 43 - 46 |
| $Na_2O$ | 3 - 5 |
| MgO | 2- 3.5 |
| $CaF_2$ | 3 - 5 |

and always additionally contains, going beyond the 100 % of the aforementioned mixture constituents, a further 0.5 to 11 % of $Al_2O_3$ and/or $ZrO_2$ or $TiO_2$ and $Al_2O_3$ or $TiO_2$ and $ZrO_2$ or $TiO_2$ and $ZrO_2$ and $Al_2O_3$, wherein $TiO_2$ can be between 0.5 to 5 %,
2.) the grain size distribution deviates from the normal distribution, wherein the proportion of particles is

| | |
|---|---|
| 200 - 500 micrometres | less than 10 % |
| 160 - 200 micrometres | 15 to 25 % |
| 125 - 160 micrometres | 40 to 50 % |
| 100 - 125 micrometres | 20 to 30 % |
| smaller than 100 micrometres | |
| less than 20 % | |
| of that smaller than 10 micrometres | |
| less than 0.1 %, and | |

3.) the radius deviations from the ball shape for socket and head are $\leq 30 \, \mu m$.

**2.** Joint endoprosthesis according to claim 1, characterised in that the inorganic composite component contains 1 to 20 parts by mass in % of secondary crystal phases, wherein the secondary crystal phases are selected from the group comprising feldspars, alpha-tricalcium phosphate, $CaTiSiO_5$, $CaZrSi_4O_{12}$ and rutile.

**3.** Joint endoprosthesis according to claim 2, characterised in that the inorganic composite component contains 5 to 20; preferably 8 to 15 % of secondary crystal phases.

4. Joint endoprosthesis according to claim 1, characterised in that the mixture also contains 0.5 to 10 % $ZrO_2$.

5. Joint endoprosthesis according to claim 1, characterised in that the mixture also contains 0.5 to 5 % $TiO_2$ and furthermore $Al_2O_3$ and/or $ZrO_2$ to a total of 10 %.

6. Joint endoprosthesis according to claim 1, characterised in that the mixture also contains 0.5 to 10 % $Al_2O_3$.

7. Joint endoprosthesis according to claim 1, characterised in that the mixture also contains 0.5 to 10 % $ZrO_2$ and $Al_2O_3$.

8. Process for producing a joint endoprosthesis with $Al_2O_3$ ceramic head and a composite socket by
   a) melting an inorganic mixture to form a ceramic, glass ceramic or sintered glass ceramic material,
   b) cooling and comminuting the crystalline material with apatite and/or wollastonite crystal phases,
   c) introducing the crystalline material having a defined grain size distribution into a polymer matrix based on polyurethane or epoxide,
   d) curing the matrix in a socket mould,
   e) and combining with an $Al_2O_3$ ceramic head, the external radius of which is designed so that the difference between internal radius of the highly polished socket and external radius of the head is in the range from 25 to 350 micrometres,
   characterised in that
   A) the mixture has the following composition (parts by mass in %)

| | |
|---|---|
| CaO | 31 - 34 |
| $P_2O_5$ | 10 - 12 |
| $SiO_2$ | 43 - 46 |
| $Na_2O$ | 3 - 5 |
| MgO | 2 - 3.5 |
| $CaF_2$ | 3 - 5 |

and always additionally contains, going beyond the 100 % of the aforementioned mixture constituents, a further 0.5 to 11 % of $Al_2O_3$ and/or $ZrO_2$ or $TiO_2$ and $Al_2O_3$ or $TiO_2$ and $ZrO_2$ or $TiO_2$ and $ZrO_2$ and $Al_2O_3$, wherein $TiO_2$ can be between 0.5 to 5 %,
B) the material thus produced having a grain size distribution of

| | |
|---|---|
| 200 - 500 micrometres | less than 10 % |
| 160 - 200 micrometres | 15 to 25 % |
| 125 - 160 micrometres | 40 to 50 % |
| 100 - 125 micrometres | 20 to 30 % |
| smaller than 100 micrometres less than 20 %, of that smaller than 10 micrometres less than 0.1 %, | |

is distributed in the polymer matrix,
C) and the radius deviation from the ball shape both for the socket and also the head are $\leq$ 30 $\mu$m.

9. Process according to claim 8, characterised in that the mixture has the following composition in mass %

| | |
|---|---|
| CaO | 31 - 34 |
| $P_2O_5$ | 10 - 12 |
| $SiO_2$ | 43 - 46 |
| $Na_2O$ | 3 - 5 |
| MgO | 2 - 3.5 |
| $CaF_2$ | 3 - 5 |

and always additionally contains, going beyond the 100 mass % of the aforementioned mixture constituents, a further 5 to 10 mass % of $Al_2O_3$ and/or $ZrO_2$ or $TiO_2$ and $Al_2O_3$ or $TiO_2$ and $ZrO_2$ or $TiO_2$ and $ZrO_2$ and $Al_2O_3$, wherein $TiO_2$ can be between 0.5 to 5 mass %.

10. Process according to claim 9, characterised in that the mixture also contains 0.5 to 5 % $TiO_2$ and furthermore $Al_2O_3$ and/or $ZrO_2$ to a total of 10 %.

11. Process according to claim 9, characterised in that $Al_2O_3$ and/or $ZrO_2$ is also added to the mixture in the same or different quantities within the limits from 0.5 to 10 mass %.

12. Process according to claim 8, in which a polyurethane, produced from a polyol or a mixture of different polyols and an aromatic, aliphatic or alicyclic diisocyanate, is used as polymer matrix, or in which the polymer matrix is formed from an epoxy resin and a curing agent, wherein the inorganic component is introduced into the resin-curing agent mixture, characterised in that the proportion of functional groups is the same or greater than 10 %.

13. Process according to claim 12, characterised in that the glass temperature of the polymer matrix is above body temperature.

14. Process according to claim 12, characterised in that when the polymer matrix is polyurethane the polyfunctionality is between 2.1 and 4.0, preferably 3.3 and 3.6.

15. Process according to claim 8 to 14, characterised in that the proportion of inorganic material is in the range from 20 to 60 parts by mass in %, based on the total composite mass.

16. Process according to claim 8, characterised in that the gelling time of the polymer component is at least 3 minutes.

## Revendications

1. Prothèse interne à rotule à tête en matière céramique $Al_2O_3$ et à cupule en matière composite, dont le composant inorganique présente des phases cristallines d'apatite et/ou de wollastonite, et dont le composant organique est un polymère réticulé à base de polyuréthane ou d'époxyde, dans laquelle le composant inorganique du composite est une céramique, une vitrocéramique ou une vitrocéramique frittée, et le matériau cuit est réparti en particules avec répartition de tailles de grain définie dans la cupule en matière composite, la différence entre le rayon intérieur de la cupule et le rayon extérieur de la tête se situant entre 25 et 350 micromètres et la cupule et la tête présentant pour l'essentiel une forme sphérique idéale,
caractérisée en ce que:
    1.) la céramique, la vitrocéramique ou la vitrocéramique frittée présente avant cuisson la composition de mélange (part de masse en %):

| CaO | 31 à 34 |
|------|---------|
| $P_2O_5$ | 10 à 12 |
| $SiO_2$ | 43 à 46 |
| $Na_2O$ | 3 à 5 |
| MgO | 2 à 3,5 |
| $CaF_2$ | 3 à 5 |

et contient de plus toujours, sortant des 100 % des constituants du mélange précités de 0,5 à 11 % de $Al_2O_3$ et/ou $ZrO_2$ ou de $TiO_2$ et $Al_2O_3$ ou de $TiO_2$ et $ZrO_2$ ou de $TiO_2$ et $ZrO_2$ et $Al_2O_3$, $TiO_2$ pouvant faire entre 0,5 et 5 %,

2.) la répartition des tailles de grains diverge par rapport à la répartition de Gauss, la part des particules se quantifiant ainsi:

| 200 à 500 micromètres | inférieur 10 % |
|------------------------|-----------------|
| 160 à 200 micromètres | 15 à 25 % |
| 125 à 160 micromètres | 40 à 50 % |
| 100 à 125 micromètres | 20 à 30 % |
| inférieur à 100 micromètres | inférieur à 20 % |
| dont inférieur à 10 micromètres | inférieur à 0,1 % |

3.) les écarts de rayons de la forme sphérique pour la cupule et la tête sont ≤ à 30 $\mu$m.

2. Prothèse interne à rotule selon la revendication 1, caractérisée en ce que le composant du composite inorganique contient des phases cristallines secondaires dans une quantité de 1 à 20 parts de masse en %, les phases cristallines secondaires étant choisies dans le groupe des feldspaths, phosphate d'alpha-tricalcium, $CaTiSiO_5$, $CaZrSi_4O_{12}$ et rutile.

3. Prothèse interne à rotule selon la revendication 2, caractérisée en ce que le composant du composite inorganique contient des phases cristallines secondaires dans une quantité de 5 à 20 %, de préférence 8 à 15 %.

4. Prothèse interne à rotule selon la revendication 1, caractérisée en ce que le mélange contient en plus du $ZrO_2$ dans une quantité de 0,5 à 10 %.

5. Prothèse interne à rotule selon la revendication 1, caractérisée en ce que le mélange contient en plus 0,5 à 5 % de $TiO_2$ et en outre $Al_2O_3$ et/ou $ZrO_2$ jusqu'à un total de 10 %.

6. Prothèse interne à rotule selon la revendication 1, caractérisée en ce que le mélange contient en plus de l'$Al_2O_3$ dans une quantité de 0,5 à 10 %.

7. Prothèse interne à rotule selon la revendication 1, caractérisée en ce que le mélange contient en plus $ZrO_2$ et $Al_2O_3$ dans une quantité de 0,5 à 10 %.

8. Procédé de fabrication d'une prothèse interne à rotule avec tête en matière céramique $Al_2O_3$ et d'une cupule en matière composite par:

a) fusion d'un mélange inorganique en un matériau céramique, vitrocéramique ou vitrocéramique fritté,

b) refroidissement et broyage du matériau cristallin avec des phases cristallines d'apatite et/ou de wollastonite,

c) incorporation du matériau cristallin présentant une répartition des tailles de grain définie dans une matrice polymère à base de polyuréthane ou d'époxyde,

d) durcissement de la matrice en forme de cupule,

e) et combinaison avec une tête en matière céramique $Al_2O_3$, dont le rayon extérieur est configuré de sorte que la différence entre le rayon intérieur de la cupule polie miroir et le rayon extérieur de la tête se trouve dans la limite de 25 à 350 micromètres;

caractérise en ce que :

A) le mélange présente la composition suivante (part de masse en %)

| | |
|---|---|
| CaO | 31 à 34 |
| $P_2O_5$ | 10 à 12 |
| $SiO_2$ | 43 à 46 |
| $Na_2O$ | 3 à 5 |
| MgO | 2 à 3,5 |
| $CaF_2$ | 3 à 5 |

et contient de plus toujours, sortant des 100 % des constituants du mélange précités de 0,5 à 11 % de $Al_2O_3$ et/ou $ZrO_2$ ou de $TiO_2$ et $Al_2O_3$ ou de $TiO_2$ et $ZrO_2$ ou de $TiO_2$ et $ZrO_2$ et $Al_2O_3$, $TiO_2$ pouvant faire entre 0,5 et 5 %
B) le matériau ainsi fabriqué avec une répartition des tailles de grain de

| | |
|---|---|
| 200 - 500 micromètres | inférieur à 10 % |
| 160 - 200 micromètres | 15 à 25 % |
| 125 - 160 micromètres | 40 à 50 % |
| 100 - 125 micromètres | 20 à 30 % |
| inférieur à 100 micromètres dont | inférieur à 20 %, |
| inférieur à 10 micromètres | inférieur à 0,1 % |

est réparti dans la matrice polymère,
C) et l'écart de rayon de la forme sphérique aussi bien de la cupule que de la sphère est ≤ à 30 $\mu$m.

9. Procédé selon la revendication 8, caractérisé en ce que le mélange présente la composition suivante en % de masse

| | |
|---|---|
| CaO | 31 à 34 |
| $P_2O_5$ | 10 à 12 |
| $SiO_2$ | 43 à 46 |
| $Na_2O$ | 3 à 5 |
| MgO | 2 à 3,5 |
| $CaF_2$ | 3 à 5 |

et contient de plus toujours, sortant des 100 % des constituants du mélange précités de 5 à 10 % de $Al_2O_3$ et/ou $ZrO_2$ ou de $TiO_2$ et $Al_2O_3$ ou de $TiO_2$ et $ZrO_2$ ou de $TiO_2$ et $ZrO_2$ et $Al_2O_3$, $TiO_2$ pouvant faire entre 0,5 et 5 %.

10. Procédé selon la revendication 9, caractérisé en ce que le mélange contient en plus 0,5 à 5 % de $TiO_2$ et en outre $Al_2O_3$ et/ou $ZrO_2$ jusqu'à un total de 10 %.

11. Procédé selon la revendication 9, caractérisé en ce qu'au mélange est ajouté $Al_2O_3$ et/ou $ZrO_2$ en parts égales ou différentes dans la limite de 0,5 à 10 % de la masse.

12. Procédé selon la revendication 8, dans lequel un polyuréthane est utilisé comme matrice polymère, fabriqué à partie d'un polyol ou d'un mélange de différents polyols et d'un diisocyanate aromatique, aliphatique ou alicyclique, ou dans lequel la matrice polymère est formée d'une résine époxy et d'un durcisseur, le composant inorganique étant introduit dans le mélange résine-durcisseur, caractérisé en ce que la part des groupes fonctionnels est égale ou supérieure à 10 %.

13. Procédé selon la revendication 12, caractérisé en ce que la température de vitrification de la matrice polymère est supérieure à la température du corps.

**14.** Procédé selon la revendication 12, caractérisé en ce que en présence d'un polyuréthane comme matrice polymère, la polyfonctionnalité se situe entre 2,1 et 4,0, de préférence entre 3,3 et 3,6.

**15.** Procédé selon l'une quelconque des revendications 8 à 14, caractérisé en ce que la part du matériau inorganique se situe entre 20 à 60 de parts de masse en %, par rapport à la masse de composite totale.

**16.** Procédé selon la revendication 8, caractérisé en ce que le temps de gélification du composant polymère fait au moins 3 minutes.